# EUROPÄISCHE PATENTSCHRIFT

(11) **EP 3 220 858 B1**
(45) Veröffentlichungstag und Bekanntmachung des Hinweises auf die Patenterteilung: **27.05.2020**
(21) Anmeldenummer: 15801370.6
(22) Anmeldetag: 19.11.2015
(51) Int. Cl.: A61F 5/56, A61C 19/045, A61B 5/00

(54) **VERFAHREN ZUM ANFERTIGUNG EINER PROTRUSIONSSCHIENE UND PROTRUSIONSSHIENE**
METHOD FOR PRODUCING A MANDIBULAR ADVANCEMENT SPLINT AND MANDIBULAR ADVANCEMENT SPLINT
PROCÉDÉ POUR LA FABRICATION D'UNE ORTHÈSE D'AVANCÉE MANDIBULAIRE ET ORTHÈSE D'AVANCÉE MANDIBULAIRE

(30) Priorität: 21.11.2014 DE 102014117080
(43) Veröffentlichungstag der Anmeldung: 27.09.2017
(73) Patentinhaber: Hicat GmbH, 53177 Bonn (DE)
(72) Erfinder: GRÜNBERG, Daniel, 53179 Bonn (DE); HANSSEN, Nils, 53125 Bonn (DE); HEY, Joachim, 53639 Königswinter (DE); KUSCH, Jochen, 53343 Wachtberg (DE)
(74) Vertreter: Braun-Dullaeus Pannen Emmerling Patent- & Rechtsanwaltspartnerschaft mbB
(86) Internationale Anmeldenummer: PCT/EP2015/077147
(87) Internationale Veröffentlichungsnummer: WO 2016/079257

(56) Entgegenhaltungen:
- EP-A1- 2 363 066
- US-A1- 2005 028 826
- US-A1- 2007 224 567
- US-A1- 2010 145 898
- US-A1- 2012 115 107
- US-A1- 2013 112 210

## Beschreibung

Die Erfindung betrifft ein Verfahren zur Anfertigung einer Protrusionsschiene umfassend eine obere Teilschiene zur Verbindung mit dem Oberkiefer und eine untere Teilschiene zur Verbindung mit dem Unterkiefer eines Patienten, wobei Führungsmittel zur gegenseitigen Führung der beiden Teilschienen während einer zubeißenden Bewegung vorgesehen sind, die den Unterkiefer über eine Führungsbahn in eine vorbestimmte Therapieposition führen, wobei ein Satz patientenindividueller Daten des Kiefergelenkapparates aufgenommen wird. Die Erfindung betrifft auch die Prutrusionsschiene selber und ein Verfahren zur Ermittlung der Therapieposition.

Solche Protrusionsschienen werden von einer Person im Mund getragen und dienen dazu, insbesondere im Schlaf den Unterkiefer der Person gegenüber einer Referenzposition weiter nach vorne zu verlagern. Unter der Referenzposition kann im Rahmen der vorliegenden Erfindung die habituelle Position verstanden werden. Damit soll insbesondere das gesundheitsschädliche oder störende Schnarchen während des Schlafens verhindert werden. Solche Protrusionsschienen umfassen eine obere Teilschiene zur Verbindung mit dem Oberkiefer der Person und eine unteren Teilschiene zur Verbindung mit dem Unterkiefer der Person. Diese Teilschienen weisen Anlageflächen für die jeweiligen Zähne und/oder Kieferbereiche auf. Die beiden Teilschienen sind grundsätzlich innerhalb gewisser Grenzen bewegbar zueinander gehalten. Zur Beaufschlagung der Kiefer in die gewünschte Kieferstellung sind Führungsmittel an beiden Teilschienen vorgesehen, die die Teilschienen in eine entsprechende Relativstellung zueinander beaufschlagen.

Grundsätzlich gibt es dabei unterschiedliche Arten von Therapieschienen, die eine z.B. anteriore Verschiebung des Unterkiefers bewirken, also den Unterkiefer im Vergleich zum Oberkiefer nach vorne verschieben, wodurch eine Protrusionsstellung der Kiefer erreicht wird.

Die DE 20 2010 006 293 U1 offenbart eine Therapieschiene in einer ersten Art. Die dort gezeigte Therapieschiene umfasst eine Oberkieferschiene und eine Unterkieferschiene mit jeweiligen Führungsflächen. Die Führungsflächen sind derart ausgebildet, dass bei einem Absinken des Unterkiefers die beiden Führungsflächen aneinander gleiten und dabei eine anteriore Verschiebung des Unterkiefers bewirken. Sobald sich der Kiefer, beispielsweise durch Erschlaffung der Muskulatur im Schlaf, öffnet, erfolgt die Beaufschlagung des Unterkiefers nach vorne.

Bei dieser Schienenart wird folglich der Kiefer bei der Nutzung umso weiter nach vorne beaufschlagt, je größer der Kiefer geöffnet ist. Damit wird dem Umstand Rechnung getragen, dass der Condylus des Kiefergelenks sich bei einer geöffneten Kieferstellung nicht nur gegenüber der Fossa verdreht, sondern sich auch relativ zur Fossa nach unten verlagert. Somit vergrößert sich grundsätzlich der Abstand des Condylus von der Gelenkbahn der Fossa, wodurch der Condylus einen größeren Freiraum in anteriorer Richtung erhält. In dieser geöffneten Stellung hat der Unterkiefer nun mehr anterioren Freiraum; der Unterkiefer kann also weiter nach vorne verlagert werden, je weiter er sich öffnet. Die Schienen dieser Art sind nicht von der vorliegenden Erfindung umfasst.

Daneben gibt es Schienen in einer zweiten Art, auf die sich die vorliegende Erfindung bezieht. Diese Schienen unterscheiden sich von den Therapieschienen der ersten Art in der Art und Weise, wie der Vorschub erzeugt wird. Bei den Protrusionsschienen dieser zweiten Art sind die Führungsmittel derart eingerichtet, dass der Unterkiefer anterior verlagert wird, wenn die Kiefer aufeinander zu bewegt werden. Der größte Grad der Protrusion wird folglich im Schlussbiss erreicht. Unter dem Begriff Schlussbiss wird im Rahmen der vorliegenden Erfindung eine solche Kieferstellung verstanden, bei die Kiefer derart nahe aufeinander zu bewegt sind, dass die beiden Teilschienen miteinander in Anschlag sind und eine weitere Schließbewegung der beiden Kiefer aufeinander zu verhindern.

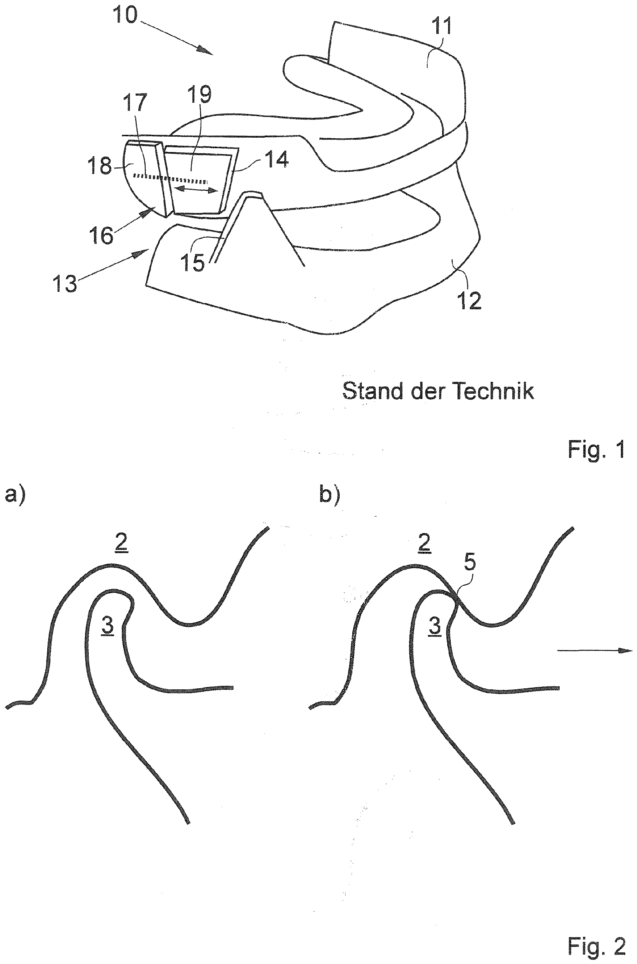

Bei diesen Therapieschienen der zweiten Art wird folglich bei geschlossener oder nahezu geschlossener Kieferstellung der größte Vorschub erzielt; bei der Therapieschiene der ersten Art vergrößert sich der Vorschub mit der Öffnung des Kieferstellung während der Nutzung. Eine bekannte Therapieschiene der zweiten Art, von der die Erfindung ausgeht, ist in der Figur 1 gezeigt und wird im Rahmen der Figurenbeschreibung unten noch näher erläutert.

Nachteilig bei solchen Therapieschienen der zweiten Art ist allerdings, dass die Protrusion nur begrenzt eingestellt werden kann, da im Falle einer großen axialen Verschiebung des Unterkiefers der Condylus recht schnell in Anschlag mit der Fossa gelangen kann. Wenn das Kiefergelenk dauerhaft in eine solch ungünstige Stellung beaufschlagt wird, kann dies Schäden am Kiefergelenksapparat hervorrufen. US 2005/028826 A1 offenbart ein Verfahren zur Anfertigung einer Protrusionsschiene. US 2012/115107 A1 offenbart ein Verfahren zur Anfertigung einer Orthese zur Behandlung von Schlafapnoe, wobei ein Satz patientenindividueller Daten des Kiefergelenkapparates aufgenommen wird.

Es ist daher Aufgabe der vorliegenden Erfindung, ein Verfahren zur Anfertigung einer Protrusionsschiene, eine Protrusionsschiene und ein Verfahren zur Ermittlung einer Therapieposition (im Folgenden gilt dies auch für eine Schar von Therapiepositionen) bereitzustellen, in der ein möglichst hoher Grad an Protrusion auch bei Vorliegen des Schlussbisses vorliegt, der aber dennoch für die Person angenehm und insbesondere nicht gesundheitsschädlich ist. Die der Erfindung zugrunde liegende Aufgabe wird gelöst durch ein Verfahren nach Anspruch 1, die Protrusionsschiene nach Anspruch 5 und das Verfahren nach Anspruch 10. Bevorzugte Ausgestaltungen ergeben sich aus der Beschreibung.

Der Kern der Erfindung liegt darin, eine Protrusionsschiene herzustellen, deren untere Teilschiene sich gegenüber der oberen Teilschiene entlang einer Führungsbahn zwangsbewegt, die aus den patientenindividuellen Daten und anhand mindestens einer daraus sich ergebenden Therapieposition bestimmt wird. Dazu wird aus den patientenindividuellen Daten vorteilhafterweise der Kurvenverlauf einer Gelenkbahn zwischen dem Gelenkkopf des Unterkiefers und der Gelenkgrube des Oberkiefers ermittelt wird, dass aus dem Verlauf der Gelenkbahn die Führungsbahn bestimmt wird und dass die Führungsmittel entsprechend der Führungsbahn angefertigt werden. Dementsprechend werden bei der erfindungsgemäßen Protrusionsschiene die Führungsmittel auf einer Führungsbahn geführt, die sich aus dem Kurvenverlauf einer anhand patientenindividuellen Daten ermittelten Gelenkbahn zwischen dem Gelenkkopf des Unterkiefers und der Gelenkgrube des Oberkiefers ergibt.

Ein weitere erfindungswesentlicher Gedanke ist, dass bei der Ermittlung der Therapieposition die Anatomie des Kiefergelenkapparates ins besonderer Form berücksichtigt wird. Das ist dadurch möglich, dass die patientenindividuellen Daten einerseits einen Satz dreidimensionaler Bilddaten des Kiefergelenks umfassen, die mit einem dreidimensionalen bildgebenden Verfahren aufgenommen wurden, und dass die patientenindividuellen Daten andererseits Bewegungsdaten aus einer Bewegungsaufzeichnung des Kieferapparates, insbesondere aus einem Kondylogramms, umfassen. Die Bewegungsdaten beschreiben die anatomisch möglichen Positionen des Kiefergelenks, wohingegen sich aus den dreidimensionalen Bilddaten die Sollbewegung ermitteln lässt. Erfindungsgemäß werden die dreidimensionalen Bilddaten mit den Bewegungsdaten zur Generierung eines simulierten Bewegungsablaufes der Kieferbewegung des Patienten im Raum der dreidimensionalen Bilddaten vereint, so dass sich aus dem simulierten Bewegungsablauf die Therapieposition oder die Schar von Therapiepositionen bestimmen lässt.

Wie bereits einleitend dargestellt, gibt es einen Zusammenhang zwischen der maximal erreichbaren Protrusion und dem Öffnungswinkel der Kiefer derart, dass eine höhere Protrusion erreichbar ist, je größer der Kieferöffnungswinkel ist. Gleichzeitig aber ist es erwünscht, den Kiefer möglichst geschlossen zu halten, aber dennoch eine möglichst hohe Vorverlagerung des Unterkiefers einzustellen. Dadurch, dass nun die Anatomie des Kiefergelenkapparates bei der Erzeugung der Therapieposition berücksichtigt wird, kann eine Optimierung dahingehend erfolgen, dass die Protrusion möglichst groß ist, bei zugleich möglichst geringem Kieferöffnungswinkel.

Zum einen werden patientenindividuelle Bilddaten des Kiefergelenkapparates, insbesondere des Kiefergelenks, bereitgestellt, die durch anhand eines an der Person durchgeführtes bildgebendes Verfahren erzeugt wurden. Aus diesen Bilddaten werden die erforderlichen Geometriedaten extrahiert. Bei den Bilddaten kann es sich auch um einfaches Röntgenbild handeln, aus dem die Form der Gelenkbahn vorzugsweise in Seitenansicht erkennbar ist. Bevorzugt sind allerdings dreidimensionale Volumendaten, insbesondere Tomographiedaten, da sich daraus wesentliche Geometrieinformationen Form des Kiefergelenksdeutlich detaillierter erzeugen lassen. Zur Aufnahme der dreidimensionale Volumendaten eignen sich beispielsweise Cone-Beam Verfahren, CT oder MRT.

Dabei werden im Folgenden unter dem Kiefergelenkapparat solche anatomischen Gegebenheiten verstanden, die für die Bewegung der Kiefer relevant sind. Insbesondere fallen darunter die sämtliche Körperteile, die eine Relativbewegung der Kiefer zueinander unterstützen oder behindern. Insbesondere umfasst sind davon die Kiefergelenke, die Muskulatur zur Ansteuerung der Kiefer, Sehnen und Knorpel im Bereich des Kiefergelenks.

Zum anderen werden patientenindividuelle Daten einer Bewegungsaufzeichnung, insbesondere eines Kondylogramms, bereitgestellt. Aus der Bewegungsaufzeichnung lässt sich ermitteln, welche Positionen der Unterkiefer der Person bei üblicher Bewegung mit hoher Wahrscheinlichkeit einnimmt; aus diesen Positionen können die Therapiepositionen ausgewählt werden.

Aus den patientenindividuellen Daten wird vorzugsweise der Kurvenverlauf der Gelenkbahn des Kiefergelenks ermittelt. Ein besonders relevanter Wert ist dabei die mittlere Neigung der Gelenkbahn des Kiefergelenks.

Es können aus den patientenindividuellen Daten ferner Hinweise auf weitere oder andere anatomische Gegebenheit ermittelt werden, die zur Ermittlung der Therapiepositionen berücksichtigt werden. Solche anatomischen Gegebenheiten umfassen insbesondere Sehnen oder Knorpel des Kiefergelenkapparates, die grundsätzlich das Einnehmen einer bestimmten Position des Unterkiefers erschweren oder behindern.

Diese so ermittelte Therapieposition lässt sich nun bei der Herstellung einer Protrusionsschiene anwenden. Die Führungsmittel dieser Schiene umfassen dabei nun Einstellmittel, mit denen der Grad der Protrusion im Schlussbiss entsprechend der zuvor ermittelten Therapieposition eingestellt werden kann. Die Einstellmittel sind dabei in Abhängigkeit der patientenindividuellen Daten, insbesondere daraus generierter Geometriedaten des Kiefergelenkapparates, ausgestaltet.

Vorzugsweise umfassen die Einstellmittel dafür ein einstellbares Führungselement, welches in einer Verstellrichtung verlagerbar ist. Die Verstellrichtung ist in Abhängigkeit zu den Geometriedaten ausgerichtet. Je nach Verlagerung des Führungselementes erfolgt die Einstellung der Protrusion. Eine hohe Verlagerung des Führungselements in Verstellrichtung bedeutet einen großen Grad an Protrusion im Schlussbiss; eine kleine Verlagerung des Führungselements in Verstellrichtung erzeugt lediglich einen kleinen Grad an Protrusion im Schlussbiss.

Vorzugsweise ist dabei die Verstellrichtung in Abhängigkeit der Gelenkbahn ausgerichtet. Das bedeutet, dass das Führungselement während der Verlagerung in etwa der Gelenkbahn folgt. Insbesondere folgt die Verstellrichtung einer Richtung, die parallel zur mittleren Neigung der Gelenkbahn ausgerichtet ist. Dies hat nun den Vorteil, dass mit der Einstellung eines höheren Protrusionsgrades der Condylus nicht nur nach vorne verlagert wird, sondern zugleich der realen Form der Gelenkbahn folgend auch weiter nach unten verlagert wird. Zwar geht damit zugleich eine Öffnung der Kieferstellung einher; dennoch kann so patientenindividuell ein möglichst großer Vorschub bei möglichst kleiner Kieferöffnung realisiert werden.

Die Erfindung wird anhand der Figuren nachfolgend näher erläutert. Hierin zeigt
- **Figur 1**: eine Protrusionsschiene nach dem Stand der Technik;
- **Figur 2**: schematisch Teile eines Kiefergelenks bei der Verwendung der Protrusionsschiene nach Figur 1;
- **Figur 3**: die erfindungsgemäß durchgeführten Schritte zum Extrahieren von Geometriedaten des Kiefergelenks;
- **Figur 4**: schematisch eine erfindungsgemäß hergestellte Protrusionsschiene;
- **Figur 5**: Teile des Kiefergelenks bei der Verwendung der Protrusionsschiene nach Figur 4;
- **Figur 6**: schematisch eine weitere erfindungsgemäß hergestellte Protrusionsschiene mit veränderten Parametern;
- **Figur 7**: Teile des Kiefergelenks bei der Verwendung der Protrusionsschiene nach Figur 7;
- **Figur 8**: a) Ausschnitte eines Kondylogramms; b) Teile des Kiefergelenks überlagert mit dem Ausschnitt des Kondylogramms nach Figur 8a;
- **Figur 9**: vergrößert der Ausschnitt des Kondylogramms nach Figur 8a mit daraus ermittelter mittlerer Gelenkbahnneigung;
- **Figur 10**: eine Überlagerung der Darstellungen der Figuren 3b und 8a.

Figur 1 zeigt eine Protrusionsschiene nach dem Stand der Technik. Sie umfasst eine obere Teilschiene 11 und eine untere Teilschiene 12, die passgenau zu einem Kiefer bzw. zu den Zähnen einer Person ausgebildet sind. Wenn die Person diese Protrusionsschiene trägt und zubeißt, wird der Unterkiefer der Person in eine Protrusionsstellung beaufschlagt, das heißt, der Unterkiefer wird nach vorne verlagert.

Zur Einstellung dieser Protrusionsstellung sind nun Führungsmittel 13 an der Protrusionsschiene 1 vorgesehen. Diese umfassen eine obere Führungsfläche 14, die an der oberen Teilschiene 11 angebracht ist, und eine untere Führungsfläche 15, die an einer unteren Teilschiene 12 angebracht ist. Diese sind leicht von hinten/unten nach vorne/oben geneigt, so dass beim Zubeißen der Unterkiefer nach vorne beaufschlagt wird.

Der Grad der Protrusion ist bei einer solchen Schiene einstellbar. Dafür umfassen die Führungsmittel 13 Einstellmittel 16. Die Einstellmittel 16 umfassen mehrere Elemente, nämlich ein festes Stützelement 18, welches an der oberen Teilschiene 11 fest angebracht ist, sowie ein bewegliches Führungselement 19, welches über eine Einstellschraube 17 und gegebenenfalls weiteren Führungselementen verschieblich gegenüber dem festen Stützelement 18 angeordnet ist. Das zweite bewegliche Führungselement 19 weist die obere Führungsfläche 14 auf. Über die Einstellschraube 17 kann nun der Abstand des beweglichen Führungselements 19 gegenüber dem festen Stützelement 18 eingestellt werden.

Figur 2 zeigt nun schematisch ein Kiefergelenk mit der Fossa 2 und dem Condylus 3. In Figur 2a ist der Normalzustand in Okklusionsstellung ohne Protrusion der Unterkiefer. Figur 2b zeigt, dass nun der Condylus 3 durch Verwendung der Protrusionsschiene nach Figur 1 nach vorne verlagert wird. Bei einer zu großen Verlagerung allerdings stößt der Condylus an die Gelenkbahn 5 der Fossa 2 an. Dies verursacht Schmerzen und Kiefergelenksprobleme bei der Person.

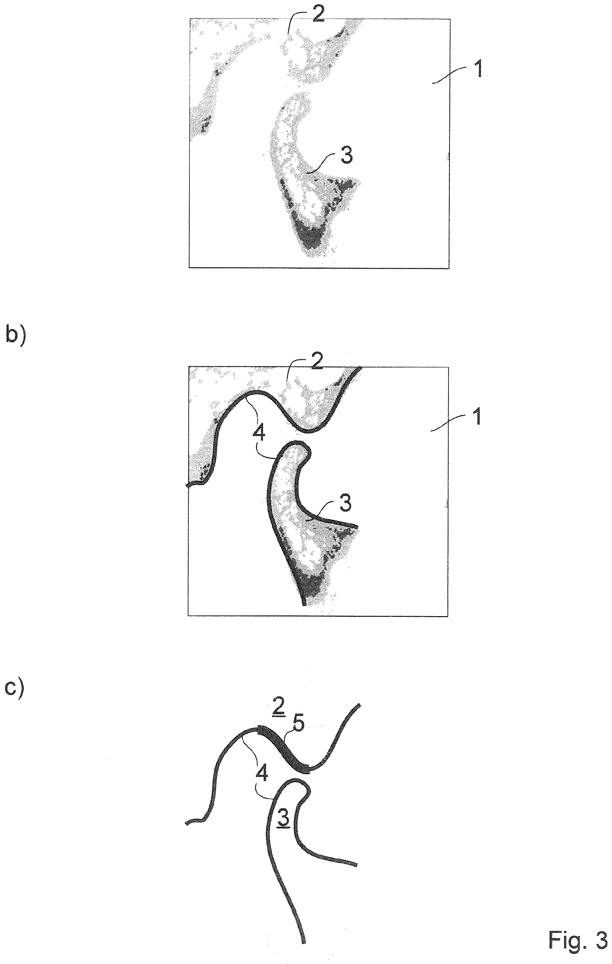

Das erfindungsgemäße Verfahren wird nun anhand der Figuren 3 bis 7 näher erläutert.

Figur 3 zeigt eine Visualisierung von Volumendaten 1 des Kiefergelenks, die mittels eines thomographischen Verfahrens erzeugt wurden. Zu erkennen ist die Fossa 2 und der Condylus 3 (Figur 3a). Nun werden aus den Bilddaten des Kiefergelenks Oberflächen 4 des Kiefergelenks extrahiert, wie aus Figur 3b zu erkennen ist. Zu erkennen ist auch die hier durch eine besonders fette Linie hervorgehobene Gelenkbahn 5, die durch eine Oberfläche der Fossa 2 gebildet ist (Figur 3c). Diese Gelenkbahn 5 hat nun für das weitere Verfahren große Bedeutung.

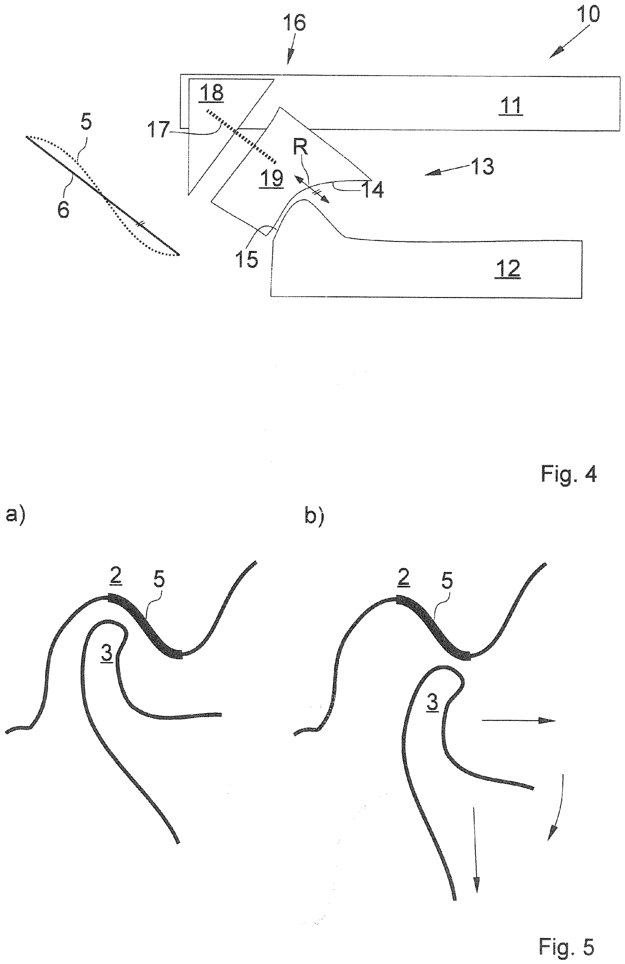

In Figur 4a wird nun aus der ermittelten Gelenkbahn 5 die mittlere Neigung 6 der Gelenkbahn 5 durch Mittelwertbildung erzeugt. Diese mittlere Gelenkbahnneigung 6 ist nun der Eingangswert für die Verstellbarkeit einer erfindungsgemäß hergestellten Protrusionsschiene 10, die schematisch in Figur 4b gezeigt ist. Diese umfasst im Wesentlichen dieselben Bauteile, wie die Protrusionsschiene 10 nach Figur 1. Allerdings ist die Verstellrichtung R, in welcher das bewegliche Führungselement 19 gegenüber dem festen Stützelement zur Einstellung des Protrusionsgrades verlagerbar ist, ausgerichtet an der mittleren Gelenkbahnneigung 6, nämlich parallel zu dieser angeordnet. Wenn nun durch die Einstellmittel 16 der Grad der Protrusion eingestellt wird, so wird nun neben dem Vorschub auch eine öffnende Beaufschlagung des Kiefers erzeugt. Dies wird auch deutlich anhand der Figur 5a, in der das Kiefergelenk in einer Okklusalstellung gezeigt ist, ohne Protrusionsschiene. Bei eingesetzter Protrusionsschiene (Figur 5b) wird zwar der Kiefer nach vorne verschoben, angedeutet durch den nach rechts weißenden Pfeil; zugleich wird aber auch der Kiefer im Schlussbiss leicht geöffnet, angedeutet durch den im Uhrzeigersinn ausgerichteten Pfeil, was automatisch eine Verlagerung des Condylus 3 nach unten bewirkt (nach unten weisender Pfeil). Es ist ersichtlich, dass durch diese Verlagerung der Condylus 3 nun der Gelenkbahn 5 weitgehend folgt. Ein Anstoßen des Condylus 3 an der Fossa 2, wie in Figur 2 noch gezeigt ist, wird so vermieden. Auch lassen sich Sehnen oder Knorpel am Kiefergelenksapparat erkennen, die das Einnehmen einer bestimmten Kieferstellung erschweren oder behindern.

Alternativ könnte die Verstellrichtung R auch einen gekrümmten Verlauf aufweisen, insbesondere um die Gelenkbahn real nachzubilden. Zusätzlich zur Einstellschraube könnte dabei eine gekrümmte Führungsschiene vorhanden sein, entlang der das Führungselement 19 geführt wird. So könnte nicht nur die mittlere Gelenkbahnneigung sondern auch Abschnitte des realen Gelenkbahnverlaufs auf die Verstellrichtung abgebildet werden.

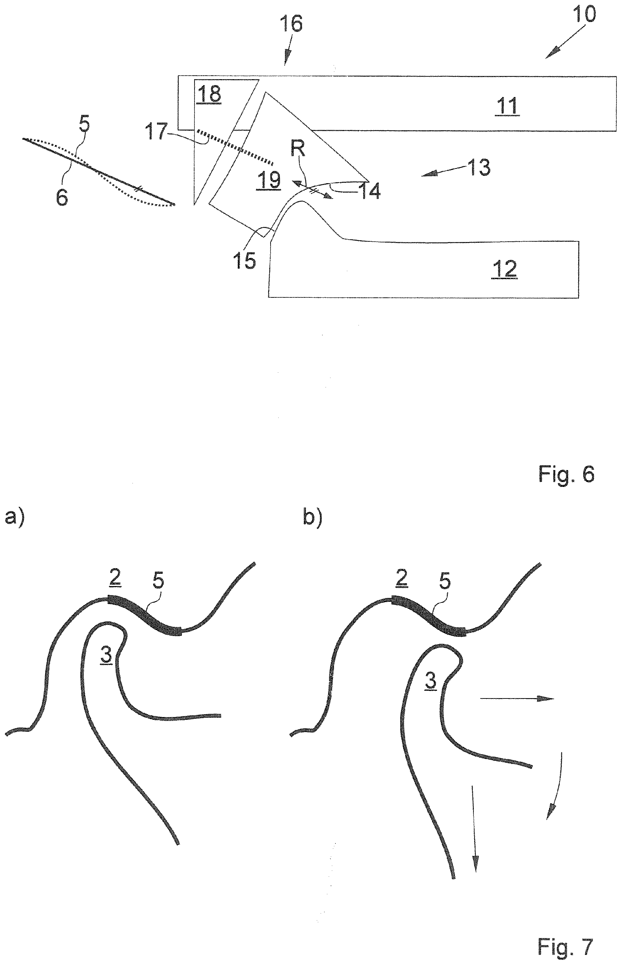

In den Figuren 6 und 7 ist nun eine alternative Ausführung der Protrusionsschiene gezeigt. Figur 6 zeigt dabei eine Protrusionsschiene mit einer flacheren Ausrichtung der Einstellschraube 17. Diese Protrusionsschiene wird angewendet bei Personen, deren Kiefergelenk eine geringere mittlere Gelenkbahnneigung aufweist. In Figur 7 ist eine entsprechende Abbildung eines solchen Kiefergelenks gezeigt. Im Vergleich zur Figur 5 ist die Gelenkbahn 5 deutlich flacher ausgerichtet.

Die Erfindung sieht folglich vor, die Verstellrichtung der Einstellmittel zur Einstellung des Grades der Protrusion unmittelbar auszurichten an der individuellen Gelenkbahn der Person. Bei Personen mit steiler Gelenkbahn muss entsprechend für die Einstellung einer Protrusion eine stärkere Öffnungsbewegung mit eingerechnet werden, was durch die steilere Anstellung der Einstellschraube 17 bzw. eine steilere Einstellung der Verstellrichtung R erreicht wird. Bei Personen mit flacherer Gelenkbahnneigung kann die Verstellrichtung R auch deutlich flacher ausgerichtet werden. Eine Vergrößerung des Grades der Protrusion geht dann mit einer geringeren geöffneten Kieferstellung einher.

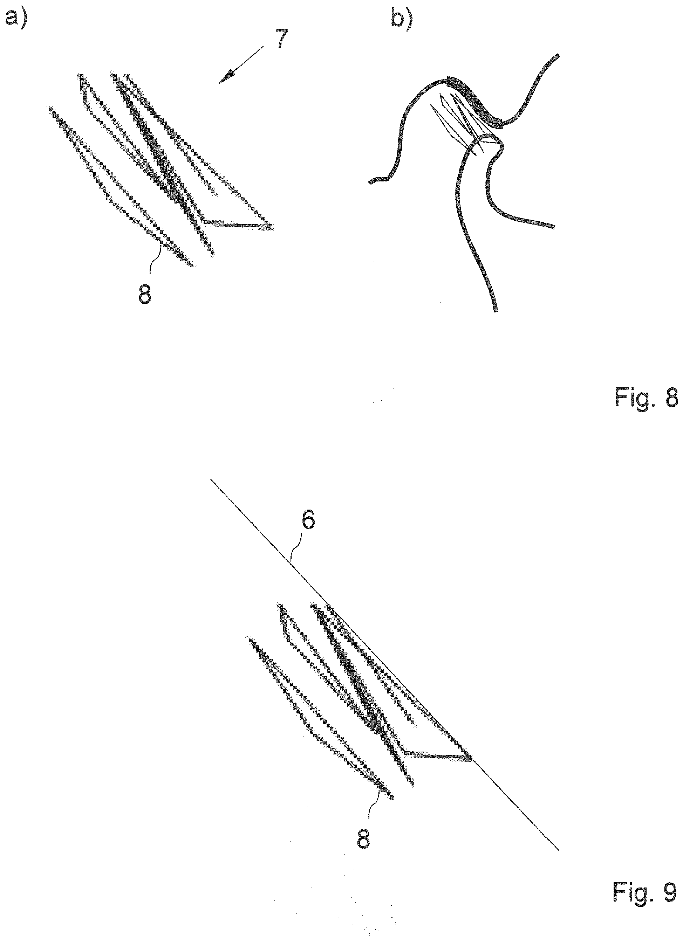

Die mittlere Gelenkbahnneigung 6 lässt sich noch durch andere Verfahren ermitteln. Figur 8a zeigt ein Kondylogramm 7 mit einer Vielzahl von Bewegungsspuren 8. Die Bewegungsspuren stehen stellvertretend für Relativpositionen des Condylus 3 gegenüber der Fossa, wie Figur 8b verdeutlichen soll. Es lassen sich so folglich Positionen ermitteln, insbesondere eine Häufigkeit von Positionen, die die Person selbständig einnimmt. Aus dieser Menge von eingenommenen Positionen lässt sich die eine Therapieposition oder die Schar von Therapieposition auswählen. Außerdem lässt sich durch Extraktion der Punkte am rechten Rand die Form der Gelenkbahn, insbesondere die mittlere Neigung 5 der Gelenkbahn 4, zumindest näherungsweise ermitteln.

Ferner lassen sich dem Kondylogramm Kieferstellungen entnehmen, die die Person meidet und daher ungeeignet sind für eine Therapieposition. Dies kann bedingt sein durch Knorpel und Sehnen, die die Einnahme einer solchen Stellung erschweren oder verhindern.

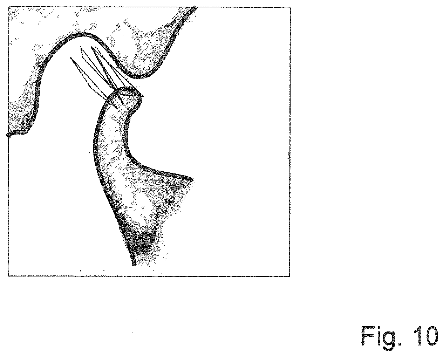

Die zwei vorgestellten Verfahren zur Ermittlung der Therapieposition können miteinander kombiniert werden. Figur 10 zeigt eine Ansicht, in der die Bilddaten mit den Kondylographiedaten überlagert sind.

### Bezugszeichenliste

- 1: 3D-Aufnahme
- 2: Fossa
- 3: Kondulus
- 4: extrahierte Oberflächen
- 5: Gelenkbahn
- 6: mittlere Gelenkbahnneigung
- 7: Ausschnitt aus Kondylogramm
- 8: Bewegunsgspuren
- 10: Protrusionsschiene
- 11: obere Teilschiene
- 12: untere Teilschiene
- 13: Führungsmittel
- 14: obere Führungsfläche
- 15: untere Führungsfläche
- 16: Einstellmittel
- 17: Einstellschraube
- 18: festes Stützelement
- 19: bewegliches Führungselement

- R: Verstellrichtung

## Patentansprüche

1. Verfahren zur Anfertigung einer Protrusionsschiene (10) umfassend eine obere Teilschiene (11) zur Verbindung mit dem Oberkiefer und eine untere Teilschiene (12) zur Verbindung mit dem Unterkiefer eines Patienten, wobei Führungsmittel (13) zur gegenseitigen Führung der beiden Teilschienen (11, 12) während einer zubeißenden Bewegung vorgesehen sind, die den Unterkiefer über eine Führungsbahn in eine vorbestimmte Therapieposition führen, wobei ein Satz patientenindividueller Daten (1, 4, 5, 6, 7, 8, 9, 20) des Kiefergelenkapparates (2, 3) aufgenommen wird,
**dadurch gekennzeichnet,**
**dass** die Führungsbahn aus den patientenindividuellen Daten (1, 4, 5, 6, 7, 8, 9) bestimmt wird und dass die Führungsmittel (13) entsprechend der Führungsbahn angefertigt werden,
wobei aus den patientenindividuellen Daten der Kurvenverlauf einer Gelenkbahn zwischen dem Gelenkkopf des Unterkiefers und der Gelenkgrube des Oberkiefers ermittelt wird und
wobei aus dem Verlauf der Gelenkbahn die Führungsbahn bestimmt wird und die Führungsmittel entsprechend der Führungsbahn angefertigt werden.

2. Verfahren nach Anspruch 1,
**dadurch gekennzeichnet,**
**dass** aus dem Kurvenverlauf der Gelenkbahn eine mittlere Neigung bestimmt wird, die auf die Führungsbahn übertragen wird.

3. Verfahren nach einem der vorherigen Ansprüche,
**dadurch gekennzeichnet,**
**dass** als patientenindividuelle Daten mit einem bildgebenden Verfahren Bilddaten (1) des Kiefergelenks (2, 3) insbesondere in verschiedenen Relativpositionen der Kiefer, aufgenommen werden.

4. Verfahren nach einem der vorherigen Ansprüche,
**dadurch gekennzeichnet,**
**dass** Bewegungsdaten (7) des Kieferapparates, insbesondere in Form eines Kondylogramms (8), aufgenommen werden und dass aus den Bewegungsdaten (7) die relevanten patientenindividuellen Daten (6) erzeugt werden.

5. Protrusionsschiene (10) angefertigt nach dem Verfahren entsprechend einem der vorherigen Ansprüche, wobei die Protrusionsschiene eine obere Teilschiene (11) und eine untere Teilschiene (12) umfasst und wobei Führungsmittel (13) zur gegenseitigen Führung der beiden Teilschienen (11, 12) während einer zubeißenden Bewegung vorgesehen sind, wobei die Führungsmittel (13) den Oberkiefer und den Unterkiefer über eine Führungsbahn in eine vorbestimmte Therapieposition führen,
**dadurch gekennzeichnet,**
**dass** die Führungsmittel (13) auf einer Führungsbahn geführt sind, die sich aus dem Kurvenverlauf einer anhand patientenindividuellen Daten (1, 4, 5, 6, 7, 8, 9, 20) ermittelten Gelenkbahn zwischen dem Gelenkkopf des Unterkiefers und der Gelenkgrube des Oberkiefers ergibt,
wobei der Kurvenverlauf einer Gelenkbahn zwischen dem Gelenkkopf des Unterkiefers und der Gelenkgrube des Oberkiefers aus den patientenindividuellen Daten ermittelt ist und
wobei die Führungsbahn aus dem Verlauf der Gelenkbahn bestimmt ist und die Führungsmittel entsprechend der Führungsbahn angefertigt sind.

6. Protrusionsschiene nach Anspruch 5,
**dadurch gekennzeichnet,**
**dass** die Führungsmittel (13) ein Einstellmittel (16) zur Einstellung des Grades der Protrusion im Schlussbiss umfassen, wobei die Einstellmittel (16) in Abhängigkeit zu den patientenindividuellen Daten ausgestaltet sind.

7. Protrusionsschiene nach Anspruch 6,
**dadurch gekennzeichnet,**
**dass** das Einstellmittel (16) ein einstellbares Führungselement (19) umfasst, das in einer Verstellrichtung (R) verlagerbar ist, wobei die Verstellrichtung in Abhängigkeit zu den patientenindividuellen Daten ausgerichtet ist.

8. Protrusionsschiene nach Anspruch 7,
**dadurch gekennzeichnet,**
**dass** die Verstellrichtung (R) in Abhängigkeit der Gelenkbahn (5) ausgerichtet ist.

9. Protrusionsschiene nach einem der Ansprüche 7 oder 8,
**dadurch gekennzeichnet,**
**dass** Verstellrichtung (R) parallel zur mittleren Neigung (6) der Gelenkbahn (5) ausgerichtet ist.

10. Verfahren nach einem der Ansprüche 1-4, wobei ein Verfahren zur Ermittlung einer Therapieposition oder einer Schar von Therapiepositionen als Grundlage für die Anfertigung einer Protrusionsschiene (10) nach dem Verfahren nach einem der Ansprüche 1 bis 4 vorgesehen ist,
**dadurch gekennzeichnet,**
**dass** patientenindividuelle Daten (1, 4, 5, 6, 7, 8, 9, 20) umfassend einerseits einen Satz dreidimensionaler Bilddaten (1) des Kiefergelenks (2, 3) mit einem dreidimensionalen bildgebenden Verfahren und umfassend andererseits Bewegungsdaten (7) aus einer Bewegungsaufzeichnung des Kieferapparates, insbesondere aus einem Kondylogramms (8), gewonnen werden, dass die dreidimensionalen Bilddaten (1) mit den Bewegungsdaten zur Generierung eines simulierten Bewegungsablaufes der Kieferbewegung des Patienten im Raum der dreidimensionalen Bilddaten (1) vereint werden und dass aus dem simulierten Bewegungsablauf die Therapieposition oder die Schar von Therapiepositionen bestimmt wird.

## Claims

1. A method for manufacturing a mandibular advancement splint (10) comprising an upper part splint (11) for connection to the upper jaw and a lower part splint (12) for connection to the lower jaw of a patient, guide means (13) being provided for mutually guiding the two part splints (11, 12) during a biting movement, which guide the lower jaw via a predetermined guide path into a predetermined therapy position, a set of individual patient data (1, 4, 5, 6, 7, 8, 9, 20) of the temporomandibular-joint apparatus (2, 3) being recorded,
**characterized**
**in that** the guide path being determined from the individual patient data (1, 4, 5, 6, 7, 8, 9) and in that the guide means (13) are manufactured in accordance with the guide path,
wherein the curve shape of a joint path between the condyle of the lower jaw and the glenoid fossa of the upper jaw is determined from the individual patient data, and
wherein the guide path is determined from the course of the joint path and the guide means are manufactured in accordance with the guide path.

2. The method according to Claim 1,
**characterized**
**in that** an average slope is determined from the curve shape of the joint path, which is transmitted to the guide path.

3. The method according to one of the preceding claims,
**characterized**
**in that** image data (1) of the temporomandibular joint (2, 3), particularly in various relative positions of the jaw, are recorded as individual patient data using an imaging method.

4. The method according to one of the preceding claims,
**characterized**
**in that** movement data (7) of the jaw apparatus are recorded, particularly in the form of a condylograph (8), and in that the relevant individual patient data (6) are created from the movement data (7).

5. A mandibular advancement splint (10) manufactured according to the method according to one of the preceding claims, the mandibular advancement splint comprising an upper part splint (11) and a lower part splint (12) and guide means (13) being provided for mutually guiding the two part splints (11, 12) during a biting movement, the guide means (13) guiding the upper jaw and the lower jaw via a guide path into a predetermined therapy position,
**characterized**
**in that** the guide means (13) are guided on a guide path, which results from the curve shape of a joint path between the condyle of the lower jaw and the glenoid fossa of the upper jaw determined on the basis of individual patient data (1, 4, 5, 6, 7, 8, 9, 20), wherein the curve shape of a joint path between the condyle of the lower jaw and the glenoid fossa of the upper jaw is determined from the individual patient data, and
wherein the guide path is determined from the course of the joint path and the guide means are manufactured in accordance with the guide path.

6. The mandibular advancement splint according to Claim 5,
**characterized**
**in that** the guide means (13) comprise an adjustment means (16) for adjusting the degree of protrusion in occlusion, wherein the adjustment means (16) are configured as a function of the individual patient data.

7. The mandibular advancement splint according to Claim 6,
**characterized**
**in that** the adjustment means (16) comprises an adjustable guide element (19), which can be displaced in a displacement direction (R), wherein the displacement direction is orientated as a function of the individual patient data.

8. The mandibular advancement splint according to Claim 7,
**characterized**
**in that** the displacement direction (R) is orientated as a function of the joint path (5).

9. The mandibular advancement splint according to one of Claims 7 or 8,
**characterized**
**in that** the displacement direction (R) is orientated parallel to the average slope (6) of the joint path (5) .

10. The method according to one of Claims 1-4, a method being provided for determining a therapy position or a collection of therapy positions as a basis for manufacturing a mandibular advancement splint (10) according to the method according to one of Claims 1 to 4,
**characterized**
**in that** individual patient data (1, 4, 5, 6, 7, 8, 9, 20), on the one hand comprising a set of three-dimensional image data (1) of the temporomandibular joint (2, 3) with a three-dimensional imaging method and on the other hand comprising movement data (7) from a movement recording of the jaw apparatus, particularly from a condylograph (8), are obtained, in that the three-dimensional image data (1) are combined with the movement data for generating a simulated movement sequence of the jaw movement of the patient in the scope of the three-dimensional image data (1), and
**in that** the therapy position or the collection of therapy positions is determined from the simulated movement sequence.

## Revendications

1. Procédé de fabrication d'une orthèse d'avancée mandibulaire (10) comprenant une partie de gouttière supérieure (11) pour la liaison avec le maxillaire supérieur et une partie de gouttière inférieure (12) pour la liaison avec le maxillaire inférieur d'un patient, sachant que des moyens de guidage (13) sont prévus pour le guidage réciproque des deux parties de gouttière (11, 12) pendant un mouvement de morsure à effectuer, qui guident le maxillaire inférieur sur une voie de guidage dans une position thérapeutique prédéfinie, sachant qu'un jeu de données individuelles propres au patient (1, 4, 5, 6, 7, 8, 9, 20) de l'appareil temporo-maxillaire (2, 3) est enregistré
**caractérisé**
**en ce que** la voie de guidage est déterminée à partir des données individuelles propres au patient (1, 4, 5, 5, 6, 7, 8, 9) et en ce que les moyens de guidage (13) sont réalisés conformément à la voie de guidage, sachant que l'allure de courbe d'un trajet articulaire entre le condyle du maxillaire inférieur et la cavité glénoïde du maxillaire supérieur est déterminée à partir des données individuelles propres au patient et sachant que la voie de guidage est déterminée à partir de l'allure du trajet articulaire et les moyens de guidage sont réalisés conformément à la voie de guidage.

2. Procédé selon la revendication 1,
**caractérisé**
**en ce qu'** à partir de l'allure de courbe du trajet articulaire une pente moyenne est déterminée, qui est transmise à la voie de guidage.

3. Procédé selon l'une quelconque des revendications précédentes,
**caractérisé**
**en ce que** des données-images (1) de l'articulation temporo-maxillaire (2, 3), en particulier dans différentes positions relatives des mâchoires, sont enregistrées en tant que données individuelles propres au patient avec un procédé d'imagerie.

4. Procédé selon l'une quelconque des revendications précédentes,
**caractérisé**
**en ce que** les données de mouvement (7) de l'appareil temporo-maxillaire sont enregistrées, en particulier sous la forme d'un condylogramme (8) et en ce que les données individuelles importantes propres au patient (6) sont produites à partir des données de mouvement (7) .

5. Orthèse d'avancée mandibulaire (10) réalisée selon le procédé selon l'une quelconque des revendications précédentes, sachant que l'orthèse d'avancée mandibulaire comprend une partie de gouttière supérieure (11) et une partie de gouttière inférieure (12) et sachant que des moyens de guidage (13) sont prévus pour le guidage réciproque des deux parties de gouttière (11, 12) pendant un mouvement de morsure à effectuer, sachant que les moyens de guidage (13) guident le maxillaire supérieur et le maxillaire inférieur sur une voie de guidage dans une position thérapeutique prédéfinie,
**caractérisé**
**en ce que** les moyens de guidage (13) sont guidés sur une voie de guidage, qui résulte de l'allure de courbe d'un trajet articulaire déterminé à l'aide des données individuelles propres au patient (1, 4, 5, 6, 7, 8, 9, 20) entre le condyle du maxillaire inférieur et la cavité glénoïde du maxillaire supérieur, sachant que l'allure de courbe d'un trajet articulaire entre le condyle du maxillaire inférieur et la cavité glénoïde du maxillaire supérieur est déterminée à partir des données individuelles propres au patient et
sachant que la voie de guidage est définie à partir de la courbe du trajet articulaire et les moyens de guidage sont réalisés conformément à la voie de guidage.

6. Orthèse d'avancée mandibulaire selon la revendication 5,
**caractérisé**
**en ce que** les moyens de guidage (13) comprennent un moyen de réglage (16) destiné à régler le degré d'avancée mandibulaire en occlusion finale, sachant que les moyens de réglage (16) sont structurés en fonction des données individuelles propres au patient.

7. Orthèse d'avancée mandibulaire selon la revendication 6,
**caractérisé**
**en ce que** le moyen de réglage (16) comprend un élément de guidage réglable (19), qui peut être déplacé dans une direction de réglage (R), sachant que la direction de réglage est orientée en fonction des données individuelles propres au patient.

8. Orthèse d'avancée mandibulaire selon la revendication 7,
**caractérisé**
**en ce que** la direction de réglage (R) est orientée en fonction du trajet articulaire (5).

9. Orthèse d'avancée mandibulaire selon l'une quelconque des revendications 7 ou 8,
**caractérisé**
**en ce que** la direction de réglage (R) est orientée parallèlement à la pente moyenne (6) du trajet articulaire (5).

10. Procédé selon l'une quelconque des revendications 1-4, sachant qu'un procédé destiné à déterminer une position thérapeutique ou un ensemble de positions thérapeutiques est prévu en tant que base pour la fabrication d'une orthèse d'avancée mandibulaire (10) selon le procédé selon l'une quelconque des revendications 1 à 4
**caractérisé**
**en ce que** des données individuelles propres au patient (1, 4, 5, 6, 7, 8, 9, 20) comprenant d'une part un jeu de données-images tridimensionnelles (1) de l'articulation temporo-maxillaire (2, 3) sont obtenues avec un procédé d'imagerie tridimensionnel et comprenant d'autre part des données de mouvement (7) obtenues à partir d'un enregistrement de mouvement de l'appareil temporo-maxillaire, en particulier à partir d'un condylogramme (8), en ce que les données d'imagerie tridimensionnelles (1) sont réunies aux données de mouvement pour générer un cycle de mouvement simulé du mouvement maxillaire propres au patient dans l'espace des données-images tridimensionnelles (1) et en ce que la position thérapeutique ou l'ensemble des positions thérapeutiques est déterminé à partir du cycle de mouvement simulé.
